# EUROPEAN PATENT SPECIFICATION

(11) **EP 4 185 356 B1**
(45) Date of publication and mention of the grant of the patent: **28.08.2024**
(21) Application number: 21751853.9
(22) Date of filing: 23.07.2021
(51) Int. Cl.: A61M 16/06, A61M 16/08

(54) **NASAL CANNULA**
NASENKANÜLE
CANULE NASALE

(30) Priority: 24.07.2020 GB 202011539
(43) Date of publication of application: 31.05.2023
(73) Proprietor: Flexicare (Group) Limited, Mountain Ash, Mid Glamorgan CF45 4ER (GB)
(72) Inventor: POORMAND, Ghassem, London, Greater London NW1 4RD (GB); THOMAS, Tudor Norman, Mountain Ash, Mid Glamorgan CF45 4ER (GB); POORMAND, Shahrzad, Irvine, California CA 92618 (US)
(74) Representative: Mewburn Ellis LLP
(86) International application number: PCT/GB2021/051911
(87) International publication number: WO 2022/018459

(56) References cited:
- WO-A1-2009/109005
- CN-A- 102 625 720
- US-A- 4 422 456
- US-A1- 2012 318 270
- US-A1- 2018 001 045
- US-A1- 2020 101 250

## Description

### FIELD OF THE INVENTION

The present invention relates to a nasal cannula and a system including a nasal cannula.

### BACKGROUND OF THE INVENTION

High Flow Oxygen Therapy (HFOT) delivers an air/oxygen gas mix to the patient at flow rates that exceed the patient's inspiratory flow rates at various minute volumes. Historically, it has been used with High Airflow with Oxygen Entrainment (HAFOE) face masks with venturi barrels mixing with ambient room air to deliver a fixed concentration of oxygen. While it can be effective in supporting oxygenation, mask therapy can be limited by many factors including the inability the patient to eat or drink, difficulty in communicating, and feelings of claustrophobia while wearing the mask. This can lead to poor patient compliance.

Nasal cannulas provide an alternative to the use of masks. Nasal cannulas are more comfortable, less claustrophobic, and allow the patient to communicate more easily, as well as eat or drink. Even so, the gas tubes delivering air/gas to the patient can be intrusive, thereby decreasing patient comfort and convenience. WO2009/109005 A1 describes an apparatus for delivering a flow of breathable gas to a patient for the treatment of Sleep Disordered Breathing (SDB) that is less obtrusive. US2012/318270 A1 describes a nasal interface for use in a system for providing a flow of respiratory gases to a user, comprising a body having an inlet for connection to a conduit providing a flow of gases. US2018/001045 A1 describes a nasal cannula body which includes a first end rotatably coupled to the first connector. CN102625720 A describes a system for supplying ventilatory support that includes a nasal interface configured to communicate with a patient's nose while allowing the patient to breathe ambient air directly without flowing through the nasal interface. US2020/101250 A1 describes a mask body, a sealing cushion configured to seal around a patient's nose, and a breathing gas delivery inlet provided in the mask body configured to receive a breathing gas flow from a gas source.

### SUMMARY OF THE INVENTION

The invention is defined in the appended independent claim 1. Preferred embodiments are matter of the dependent claims.

A first aspect of the invention provides a nasal cannula configured to deliver airflow to a user, comprising: a manifold; a connector for attaching a gas tube to the manifold; a pair of non-sealing nasal prongs extending from the manifold for delivering a supply of gas to a user via the gas tube; wherein the connector is configured to form a swivel connection with the manifold so as to provide relative rotation between the manifold and the gas tube.

With this arrangement, the swivel connection provides some relative pivotal freedom between the manifold and the gas tube that allow the gas tube to be manoeuvred more easily, thereby helping to increase patient comfort and convenience whilst the patient wears the nasal cannula.

A non-sealing nasal cannula is a nasal cannula that does not form an air-tight seal with the nares of a patient.

A further aspect of the invention provides a system comprising: a nasal cannula according to the first aspect; and a gas supply configured to supply gas to a user via the gas tube.

The system may be a high flow oxygen therapy system.

A further aspect of the disclosure provides a nasal cannula configured to deliver airflow to a user, comprising: a manifold comprising a rigid manifold structure and a flexible manifold cover configured to cover at least a portion of the rigid manifold structure; a connector for attaching a gas tube to the manifold; a pair of non-sealing nasal prongs integrally formed with the flexible manifold cover and extending from the manifold for delivering a supply of gas to a user via the gas tube; and a pair of opposing straps extending from the manifold, wherein the straps are integrally formed with the flexible manifold cover.

Optionally, wherein the nasal cannula further comprises an elbow between the swivel connection and the gas tube.

With this arrangement, the effect of the swivel connection is increased as the distal end of the gas tube, i.e. the portion furthest from the elbow and the swivel connection, is provided a lever arm effect such that the end of the gas tube translates when the elbow is rotated. This allows the gas tube to be more easily moved to a more convenient position relative to the patient when in use.

Optionally, the elbow is configured to redirect gas flow at an angle greater than 30 degrees, preferably greater than 60 degrees, and more preferably at a substantially 90 degrees angle.

Optionally, the swivel connection is configured to provide at least 120 degrees relative rotation, preferably at least 180 degrees. By providing at least 120 degrees, or 180 degrees, of relative rotation between the gas tube and manifold, the gas tube can be moved more easily out of the way of the patient, thereby improving patient comfort.

Optionally, the swivel connection is configured to limit the relative rotation to an angle range less than 360 degrees, and preferably no more than 180 degrees. This can prevent the gas tube from being accidentally moved into a position that limits patient comfort.

Optionally, the swivel connection is configured to allow the gas tube to freely rotate relative to the manifold.

The nasal cannula of the first aspect comprises a selective retainer means configured to selectively hold the connector in a plurality of rotary positions relative to the manifold. This allows the gas tube to be held in a convenient position for the patient temporarily, until a new position is required. The selective retainer means may be a friction fit between the connector and the manifold.

Optionally, the nasal cannula is for a high flow oxygen therapy apparatus.

Optionally, the connector forms a snap-fit connection with the manifold. A snap-fit connection is quick to assemble.

Optionally, the gas tube has a substantially constant internal cross-sectional area, and preferably wherein the connector has the same internal cross-sectional area as the gas tube. I.e. the internal cross-sectional area experiences by the air flow does not vary along the length of the gas tube and/or the length of the connector through which it flows.

Optionally, the elbow between the swivel connection and the gas tube has a substantially constant internal cross-sectional area.

Optionally, the gas tube is malleable, and/or comprises a malleable member such that the gas tube is configured to be deformable and retain a given shape when the gas tube is manipulated. The malleable member may be a wire.

Optionally, the nasal cannula may comprise a support attached to the gas tube between opposing ends of the gas tube and configured to support a position of the gas tube when the gas tube is manipulated. The support may be a malleable support.

Optionally, the nasal cannula of the first aspect comprises a pair of opposing straps extending from the manifold.

Optionally, the opposing straps are each configured to releasably attach to a patient head band.

Optionally, the straps are flexible. The straps may be flexible such that they are unable to support their own weight or the weight of the remainder of the nasal cannula without significant deflection.

Optionally, the connector attaches to the manifold via an aperture formed in the manifold.

Optionally, the aperture is located on the manifold between opposing ends of the manifold.

Optionally, the aperture is located between the pair of non-sealing nasal prongs.

Optionally, the aperture is centrally located on the manifold equidistant between opposing ends of the manifold.

Optionally, the connector is configured to be inserted through the aperture.

Optionally, the manifold comprises a face mount portion configured to be placed proximate the face of the user, and the aperture is on an opposite side of the manifold to the face mount portion.

The swivel connection has an axis of rotation substantially normal to a face of the user.

Optionally, the swivel connection has an axis of rotation substantially normal to the plane of the face mount portion.

Optionally, the connection extends forward away from the face of the user, i.e. extends normal away from the face of the user.

Optionally, the manifold comprises a rigid manifold structure and a flexible manifold cover configured to cover at least a portion of the rigid manifold structure

Optionally, the swivel connection has a first connection portion formed in the rigid manifold structure and a second connection portion formed in the connector.

Optionally, wherein the non-sealing nasal prongs are integrally formed with the flexible manifold cover. With this arrangement, manufacturability is increased.

Optionally, the straps are integrally formed with the flexible manifold cover. With this arrangement, manufacturability is increased.

Optionally, the connector comprises a single gas tube port for connecting the manifold to only one gas tube at a time.

Optionally, the manifold comprises a face mount portion configured to be placed proximate the face of the user, the face mount portion being substantially planar. This can increase patient comfort.

Optionally, the nasal cannula is configured to deliver a flow rate of less than 100 Standard litres per minute, preferably less than 70 Standard litres per minute, and preferably a flow rate of between 30 and 50 Standard litres per minute.

Optionally, the swivel connection is non air-tight.

Optionally, the swivel connection is configured to allow gas leakage below 10% at flows of up to 50 Standard litres per minute. Preferably, the swivel connection is configured to allow gas leakage below 5% at flows of up to 50 Standard litres per minute

Optionally, the nasal cannula comprises a gas tube connected to the connector.

Optionally, the gas tube further comprises a clip configured for securing the gas tube, e.g. to a lanyard, or an item of clothing of a patient, or to one of the straps.

Optionally, the nasal cannula is for a High Flow Oxygen Therapy apparatus.

Optionally, the system comprising a patient head band attached to the straps or directly to the manifold.

### BRIEF DESCRIPTION OF THE DRAWINGS

Embodiments of the invention will now be described with reference to the accompanying drawings, in which:
Figure 1 shows a perspective view of a nasal cannula according to a first example;
Figure 2 shows an exploded view of the nasal cannula;
Figure 3 shows a cross-section of the nasal cannula;
Figure 4 shows a cross-section of the swivel connection;
Figure 5 shows a cross-section of an alternative example of the swivel connection;
Figure 6 shows a perspective view of a nasal cannula according to a second example;
Figure 7A shows a side view of the nasal cannula of the second example;
Figure 7B shows a top view of the nasal cannula of the second example;
Figure 8 shows an example in which the gas tube comprises a malleable member;
Figure 9 shows a first example including supports for supporting the gas tube;
Figure 10 shows a second example including supports for supporting the gas tube;
Figure 11 shows a clip configured to attach or secure the gas tube 7 to an object.

### DETAILED DESCRIPTION OF EMBODIMENT(S)

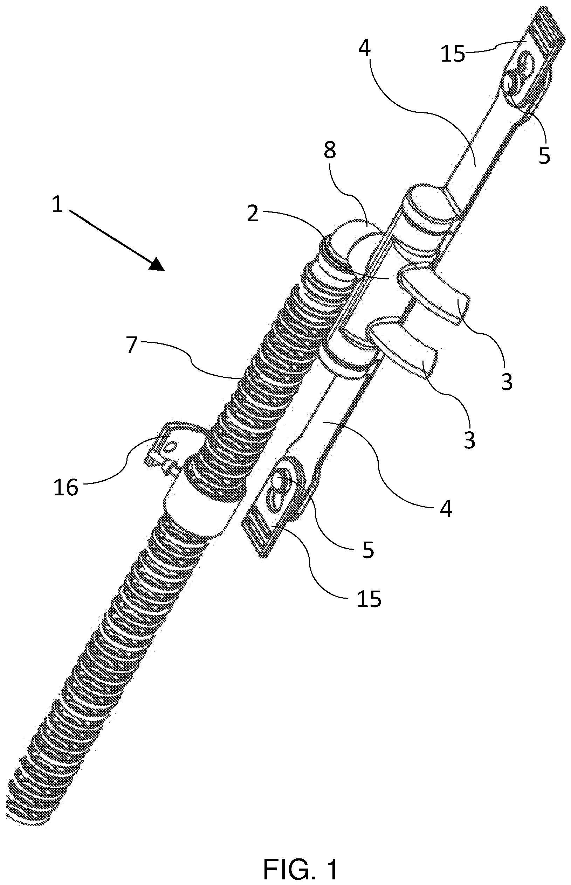

Figure 1 shows a nasal cannula 1 according to a first example. The nasal cannula 1 is used for high flow oxygen therapy (HFOT), which uses flow rates many times higher than those used typically with face masks and standard nasal cannulas. For instance, the flow rates to a mask or standard cannula may be approximately 5 Standard litres per minute (LPM) whilst the nasal cannula 1 of the present invention may provide flow rates of between 40 LPM and 70 LPM or higher, thereby creating a continuous positive airway pressure effect so that a greater area of the patent's lungs is recruited for gas exchange, further improving blood oxygenation.

The nasal cannula 1 includes a manifold 2 arranged to rest against a patient's face. A pair of non-sealing nasal prongs 3 extend from the manifold 2, which each enter a respective nasal passage (nare) of the patient.

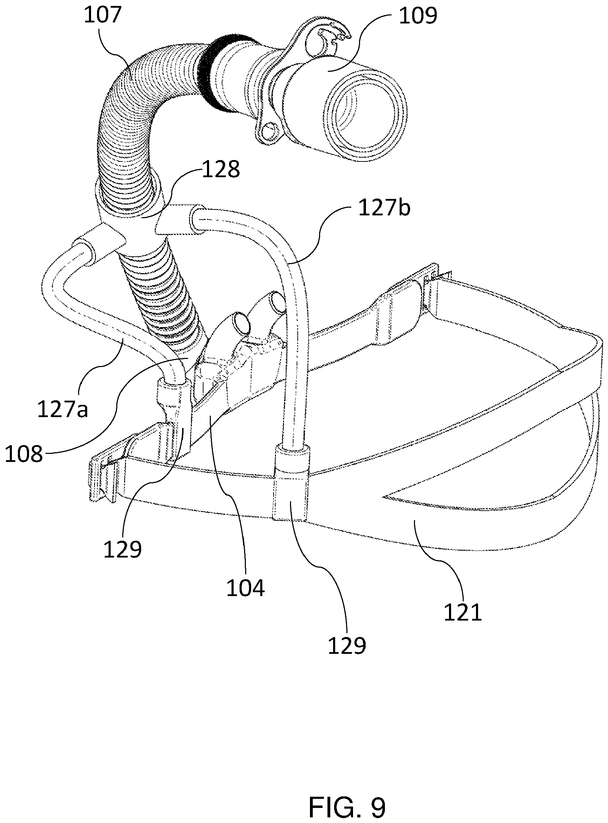

A pair of opposing straps 4 extend from the manifold 2. The straps 4 are arranged to extend around part of the patients face and include end protrusions 5 at a distal end. The end protrusions 5 provide a connection point to which corresponding clips 15 can be attached for clipping to a patient head band 121 (See Figure 9), such that different sizes and lengths of head band can be selected for a given patient or an adjustable head band can be provided.

It will be clear to the skilled person that the straps 4 may take any suitable form in order to secure the device to the patient, for example a single strap may extend from each side of the manifold 2 so as to create a loop that can be fitted over a patients head.

The straps 4 are flexible so as to conform to the shape and size of different patients, and thereby improve patient conform. The straps 4 may be flexible such that they are unable to support their own weight or the weight of the remainder of the nasal cannula 1without significant deflection. The straps 4 may be constrained by attachment to a patient head band 121 (See Figure 9), or similar, such that they are able to support the nasal cannula 1 in position on a patient. The straps 4 may comprise an elastomer, for example the straps 4 may be made of silicone.

Flexible straps 4 assist in providing equal contact pressure on the patient, or at least better distributed contact pressure. Flexible straps 4 can be particularly advantageous when performing particular oral surgeries that require the straps 4 to be oriented or otherwise manipulated at different angles, and/or when the patient has a particular facial deformity. Flexible straps 4 can also reduce the likelihood of pressure ulcers or other complications caused by prolonged pressure applied to the skin of the patient.

A gas tube 7 connects to the manifold 2 via a connector 8. The connector 8 is received inside an aperture 6 of the manifold 2 in order to form the connection between the components, as will be discussed in further detail below. In an alternative configuration the connector 8 may be received over an aperture in a projection or turret extending from the manifold 2 to form the connection.

The connector may be an elbow connector 8. The elbow connector 8 redirects the flow such that the gas flow through the aperture 6 is angled with respect to the gas flow through the gas tube 7 adjacent to the elbow connector 8. The angle of the elbow connector 8 is typically between 25 degrees and 90 degrees. In this particular example the elbow connector 8 has an angle of 90 degrees. The elbow 8 provides a sharp right angle where two respective perpendicular sections of the elbow 8 meet, although in alternative examples the elbow 8 may be swept and/or curved such that angular change is more gradual and there is no sudden angle change, thereby decreasing any pressure drop.

The aperture 6 is fluidically connected to the nasal prongs 3 such that gas may pass through the manifold 2, from a gas supply (not shown), to the nasal prongs 3.

The aperture 6 may be centrally located on the manifold 2, such that the aperture 6 is located equidistant between the nasal prongs 3, although in alternative examples the aperture can be offset such that it is further from one of the nasal prongs 3. The aperture 6 may be a single inlet aperture fluidically connecting the gas tube to the manifold.

The connection arrangement between the circular aperture 6 and the connector 8 allows the gas tube 7 to pivot about an axis of the circular aperture. This pivotal connection (alternatively referred to as a swivel connection) allows the distal end of the gas tube 7, relative to the connector 8, to be easily moved to a most convenient position relative to the patient when in use.

The connection arrangement is non air-tight, such that some air/gas is able to escape or enter through the connection. The gas leakage through the connection may be below 10% at flows of up to 50 Standard litres per minute. In alternative examples, the gas leakage may be below 5% at 50 Standard litres per minute.

The gas tube 7 connected to the connector 8 may be flexible. The gas tube may be sufficiently resilient to retain a substantially constant cross section of air/gas flow yet can bend so the gas tube can be comfortably routed to a patient's face. The gas tube 7 may be a corrugated tube, e.g. with a corrugated outer surface. The inner surface of the gas tube 7 may also be corrugated, although in alternative examples the inner and/or outer surface may be smooth.

The opposing end of the gas tube 7 to the connector 8 may be connected to a gas supply (not shown), either directly or via another gas flow path.

The nasal cannula 1 includes a clip 16 arranged on the gas tube 7 and configured to attach or secure the gas tube 7 to an object. This helps to reduce the strain on the gas tube, as well as help to more easily position and fix the gas tube in position in use. For example, the clip 16 may be a garment clip, or attachable to a garment clip, for attaching the gas tube to an item of clothing of the patient. In alternative examples, the clip 16 may be attachable to the straps, or may be attachable to a lanyard.

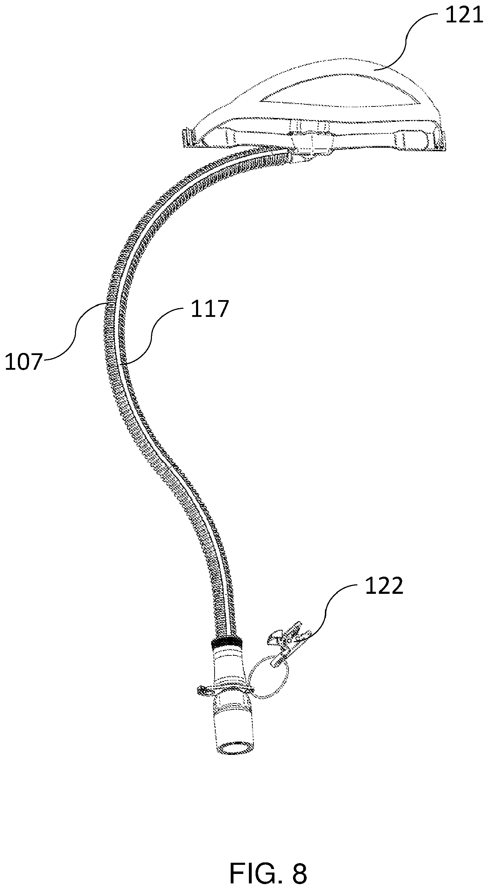

The gas tube 7 may be malleable, or comprise a malleable member 117 (as shown in Figure 8), such that the gas tube 7 is able to retain a given shape or position when manipulated (e.g. bent or twisted) into that shape or position. This allows the gas tube 7 to be positioned so as to improve patient comfort and/or clinician access. The entire length of the gas tube 7 may be malleable, or comprise a malleable member, or only a portion of the gas tube 7 may be malleable, or comprise a malleable member.

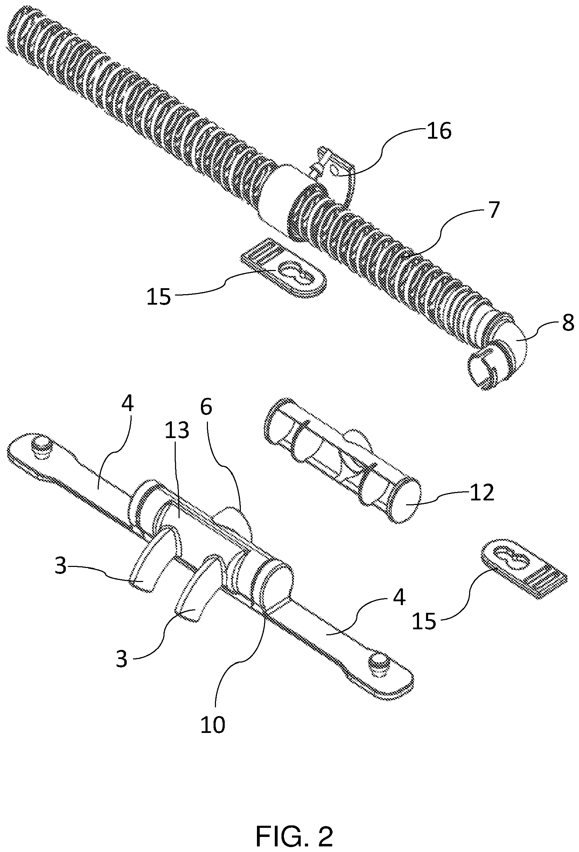

As shown in Figure 2, the manifold 2 may be a two part manifold including a rigid manifold structure 12, and a flexible manifold cover 13 arranged to fit on top of the rigid manifold structure 12. The rigid manifold structure 12 may comprise any suitably rigid material, such as plastic or metal. The flexible manifold cover 13 may comprise any suitably flexible material, such as an elastomer. In one particular example, the rigid manifold structure 12 comprises polypropylene and the flexible manifold cover 13 comprises silicone. The terms rigid and flexible are relative terms referring to rigidity of the manifold structure 12 relative to the flexibility of the manifold cover 13.

By forming part of the manifold 2 of a flexible material, i.e. the flexible manifold cover 13, the nasal prongs 3 and straps 4 can be integrally formed with the manifold 2. This construction improves manufacturability, as the nasal prongs 3 and straps 4 can be formed as part of the flexible manifold cover 13 whilst maintaining patient conform by using a relatively flexible material for the nasal prongs 3 and straps 4. In this example, the flexible manifold cover 13 substantially covers all of the rigid manifold structure 12. The flexible manifold cover 13 comprises a substantially air-tight membrane that forms an airway between the nasal prongs 3 and the gas tube 7. The rigid manifold structure 12 supports the flexible manifold cover 13 to form the shape of the manifold 2, and provides a rigid structure to which parts can be attached (e.g. the connector 8).

The manifold 2 may be substantially cylindrical in shape except for a flat or planar face mount portion 10. The face mount portion 10 may be arranged to be proximate the face of the patient in use, and contact the patients face. The face mount portion 10 provides an increased surface area to improve patient comfort in comparison to an equivalent circular or curved portion.

The straps 4 also conveniently extend from the planar face mount portion 10, providing for ease of manufacture.

The rigid manifold structure 12 provides a structural skeleton for the flexible manifold cover 13. The aperture 6 may be formed in the rigid manifold structure 12.

As previously discussed, the connection arrangement between the circular aperture 6 and the connector 8 allows the gas tube 7 to pivot about an axis of the circular aperture. This pivotal connection, or swivel connection, includes a first portion formed into the circular aperture 6 and a second portion formed into the connector 8 which allow the connector 8 to pivot relative to the manifold 2 whilst preventing any substantial translational movement at the connection between the manifold 2 and the first portion of the pivotal connection on one side, and the connector 8 and second portion of the pivotal connection on the other side.

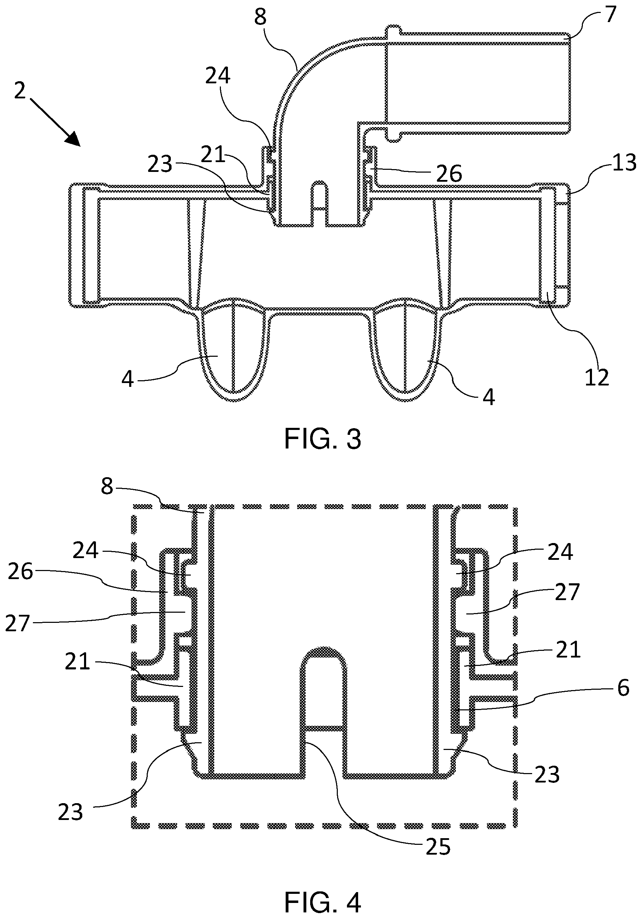

The pivotal connection is shown in further detail in Figures 3 and 4.

The first connection portion includes the circular aperture 6, as previously discussed, which may be formed in the rigid manifold structure 12. Extending from the circular aperture, into and out from the manifold 2, is a ridge 21. The ridge 21 forms a collar at the location of the aperture 6 to strengthen the aperture 6.

The second connection portion includes a first lip 23 formed into the connector 8. The first lip 23 extends out from the body of the connector 8, such that the diameter of the lip 23 is greater than the diameter of the body of the connector 8. The first lip 23 is tapered such that the diameter of the first lip 23 increases from the end of the second connection portion inwards. This helps to locate the connector 8 in the aperture 6 during assembly.

The second connection portion includes a second lip 24 formed into the connector 8. The second lip 24 extends out from the body of the connector 8, in the same way as the first lip 23, but is spaced from the first lip 23 and is not tapered such that its diameter is substantially constant.

When the connector 8 is connected to the manifold 2, the first lip 23 is positioned inside the manifold 2 and prevents the connector 8 from being withdrawn back through the aperture 6 without damaging the connector 8 and/or aperture 6. In this way, the connection is a one-way connection that is intended to remain connected once the connection is made.

When the connector 8 is connected to the manifold 2, the second lip 24 is positioned outside the manifold 2 and limits how far the connector 8 can extend into the manifold 2. The spacing between the first and second lips 23, 24 can be tailored to provide a bespoke amount of translation between the connector 8 and manifold 2, or may be designed to substantially prevent any relative translational movement when connected.

In this example, the lips 23, 24 each extend around the entire circumference of the connector 8. In alternative examples, the lips 23, 24 may extend only a portion of the distance around the circumference. In some examples, the connector 8 may include only a first lip 23 that prevents the connector 8 from exiting the aperture 6.

The initial connection between the connector 8 and the manifold 2 is facilitated by at least one slit 25 formed in the connector 8, which allows the end of the connector 8 a degree of flexibility and an ability to reduce its diameter when being inserted into the aperture 6. The connector 8 is resilient and so self-springs back to its original position once the first lip 23 (alternatively referred to as a rib, or circumferential rib) is pushed passed the aperture 6.

Such an arrangement is commonly referred to as a snap-fit connection, and allows the parts to be interlocked by pushing the parts together. A snap-fit connection allows the parts to be assembled quickly. The snap-fit connection may be a one-way snap-fit connection that does not permit ready disassembly. The interlocking may not be intended to unlock by pulling the connector 8 from the manifold 2 in normal use.

To improve the seal at the connection, the flexible manifold cover 13 is arranged to overlap part of the connection. This sealing portion 26 of the flexible manifold cover 13 is shown in Figure 4, and includes a flexible lip 27 that extends inwardly from the flexible manifold cover 13 towards the connector 8. This allows the air/gas leakage from the connection to be maintained below a target value, for example below 10% at 50 Standard litres per minute or below 5% gas loss at 30 Standard litres per minute.

The swivel arrangement between the manifold 2 and the connector 8 allows free pivotal movement between the components. In alternative examples, the swivel connection may be formed in such a way so as to limit the rotation of the connector 8 so that that the swivel connection allows a bespoke relative angular rotation between the components.

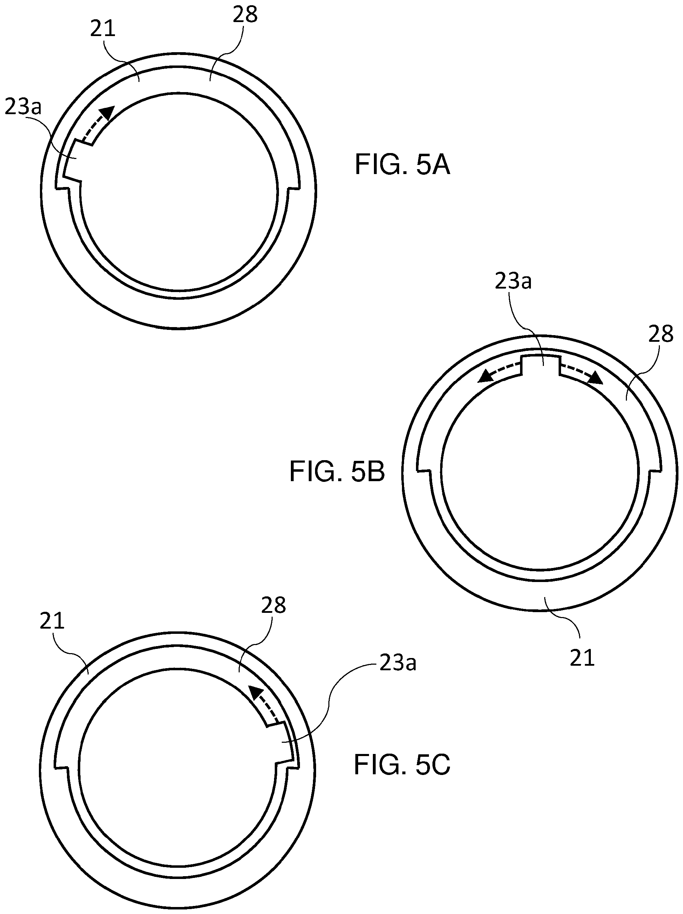

For example, Figures 5A-5C show an example in which the connector 8 comprises a part-circumferential lip 23a, i.e. the lip 23a extends from only a portion of the circumference of the connector 8. The lip 23a is placed into a groove 28 formed into the opening of the aperture 6, and the groove 28 restricts the angular movement of the lip 23a and thereby restricts the angular relative movement of the connector 8. The lip 23a of the connector 8 is thereby able to rotate from a first position (Figure 5A) through a second intermediate position (Figure 5B) to a second position (Figure 5C), but is prevented by the groove 27 from rotating any further. The lip 23a may be the first lip 23 and/or second lip 24 shown in the previous examples, or an additional lip.

In this example, the allowable range of angular rotation is approximately 170 degrees, although in alternative examples the rotation may be any angular range below 360 degrees.

In some examples, the swivel connection may be designed to allow the connector 8 to be selectively retained at an angular/rotary position, or a plurality of angular positions, relative to the manifold 2. Selectively retaining the angular position may refer to fixing the angular position in a given position until a catch or latch is released, or it may refer to a degree of resistance or torque required to rotate the connector 8 such that it will maintain its angular position until disturbed by a user. For example, a detent may be used to releasably restrict or fix the angular position, or a friction fit of the connector 8 in the aperture 6 may allow the gas tube 7 to be selectively retained in a given angular position.

The device 1 may include angular position markers to indicate a particular relative angle between the components.

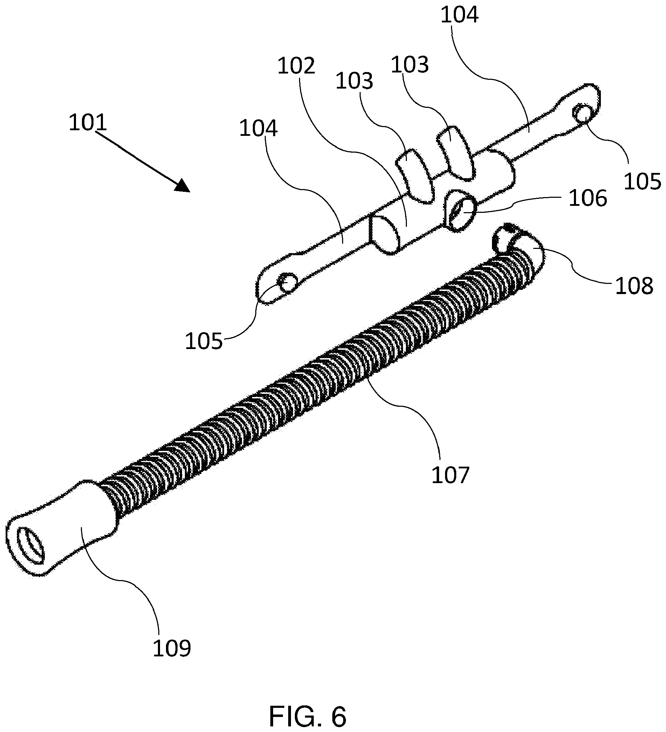

Figure 6 shows a second example of the nasal cannula 101 substantially the same as the first example. The second example differs in that the aperture 106 is on an opposite side of the manifold 102 to the face mount portion 110, as opposed to perpendicular as in the first example.

A pair of non-sealing nasal prongs 103 extend from the manifold 102 and are arranged to enter a respective nasal passage of the patient. A pair of opposing straps 104 extend from the manifold 102, and are arranged to extend around part of the patients face. The end protrusions 105, at a distal end of each strap 104, provide a connection point to which corresponding clips (e.g. clips 15 shown in relation to the first example) can be attached for clipping to a patient head band 121 or similar.

A gas tube 107 connects to the manifold 102 via a connector 108. The connector 108 may be an elbow connector 108 that redirects the air/gas flow at a substantially 90 degrees angle prior to entry into the manifold 102. The connector 108 is received inside an aperture 106 of the manifold 102. At the other end of the gas tube 107 is an end connector 109 that is connectable to a gas supply (not shown).

An advantage of the second example 101 is that the aperture 6 is opposite to the patient such that the connector extends away from the patient, which can provide an increased amount of manoeuvrability relative to the patient in some situations.

By extending away from the patient, i.e. normal to a plane of the face mount portion 110, the contact area of the nasal cannula 101 with the patient in comparison to the nasal cannula 1 shown in Figures 1 to 3 is reduced. In other words, there is no impingement of the gas tube 107 and/or connector 108 with the upper lip of the patient.

In some examples, the nasal cannula 1 shown in Figures 1 to 3 may have a gas tube 7 and/or connector 8 with a reduced diameter towards the end proximate the aperture 6, so as to reduce the contact area of the nasal cannula 1 with the upper lip of the patient. This can increase patient comfort, however it can also increase back pressure by restricting flow, thereby reducing the effectiveness of the nasal cannula 1 in providing a high flow of oxygen to a patient.

In contrast, the nasal cannula 101 shown in Figure 6 is able to provide a gas tube 107 and/or connector 108 with a constant diameter and/or constant cross-sectional area, so that back pressure is minimised, without increasing the contact area with a patient's upper lip. By having a gas tube 107 and connector 108 with a cross-sectional area that doesn't change along the path to the patient, the overall performance of the nasal cannula 101 can be increased.

Providing the aperture 106 on the opposite side of the nasal cannula 101 to the patient (i.e. opposite the face mount portion 110), such that the connector 108 extends away from the patient, also provides particular advantages when the connector 108 is an elbow connector as the gas tube 107 is able to rotate across a greater angular range.

In some examples, the swivel connection is able to fully revolve around its axis. In some examples, the allowable relative rotation may be restricted to an angle range less than 360 degrees, or less than 180 degrees, however the rotational arc can be selected to provide optimal comfort and/or performance for a particular application. For example, a particular oral surgery or facial deformity may necessitate a particular degree of angular rotatability.

Whilst the gas flow is redirected at the elbow connector 108, and within the manifold between the aperture 106 and the nasal prongs 103, the effects of this redirection are typically small compared to the effects of back pressure when reducing the size of the gas tube 107 and/or connector 108. The manufacturability of the nasal cannula 101 is also simplified by maintaining a constant diameter of the gas tube 107 and elbow connector 108.

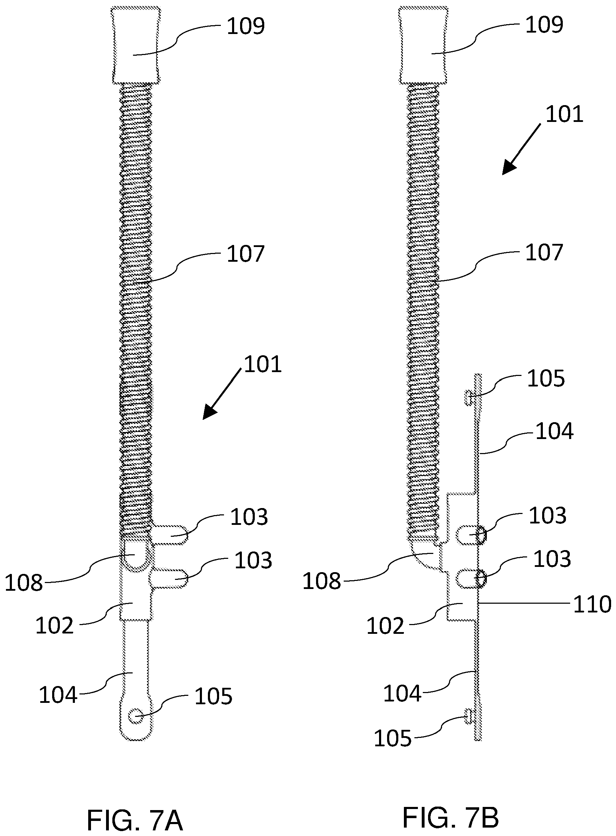

Figures 7A & 7B show a side view and top view, respectively, of the nasal cannula 101. In particular, showing the end protrusions 105 extending out from the straps 104.

Figure 8 shows an example in which the gas tube 107 comprises a malleable member 117. The malleable member 117 may be a wire. The gas tube 107 may be supported by the malleable member 117 such that manipulation of the malleable member 117 into a particular shape also results in the gas tube 107 correspondingly being manipulated into a particular shape. The malleable properties of the malleable member 117 mean that it retains a given shape or position when manipulated (e.g. bent or twisted), such that the gas tube 107 is able to retain a given shape or position when manipulated into that shape or position. This allows the gas tube 107 to be positioned so as to improve patient comfort and/or clinician access. The malleable member 117 may extend along the entire length of the gas tube 107, or the malleable member may extend along only a portion of the length of the gas tube 107 (e.g. 50% of the length). In some examples, the malleable member 117 may be a helical malleable member 117 that coils around the gas tube 107, for example on an inner or outer surface of the gas tube 107.

The nasal cannula 101 may also comprise a clip 122 on an end of the gas tube 107, so as to assist in supporting the gas tube 107. For example, figure 8 shows a crocodile clip 122 attached to an end of the gas tube 107 distal to the manifold 102. The clip 122 may attach to an item of clothing, or other item, so as to assist in securing the gas tube in position.

In some examples, the gas tube 107 may be supported by one or more supports 127a, 127b that are attached at one end to the gas tube 107. The supports 127a, 127b may be attached to the gas tube 107 at a location between the ends of the gas tube, for example between the connectors 108, 109 of the gas tube 107. The supports 127a, 127b may be attached to the gas tube 107 approximately mid-way between the connectors 108, 109.

The opposing end of the supports 127a, 127b may be attached to part of the nasal cannula 101, for example at a first connector 129. In Figure 9, a first support 127a is shown extending between the gas tube 107 and the strap 104, and a second support 127b is shown extending between the gas tube 107 and the patient head band 121, although it will be understood that the one or more supports 127a, 127b may extend from any suitable part of the nasal cannula 101, or external support structure, and from any suitable part of the gas tube 107 or connectors 108, 109.

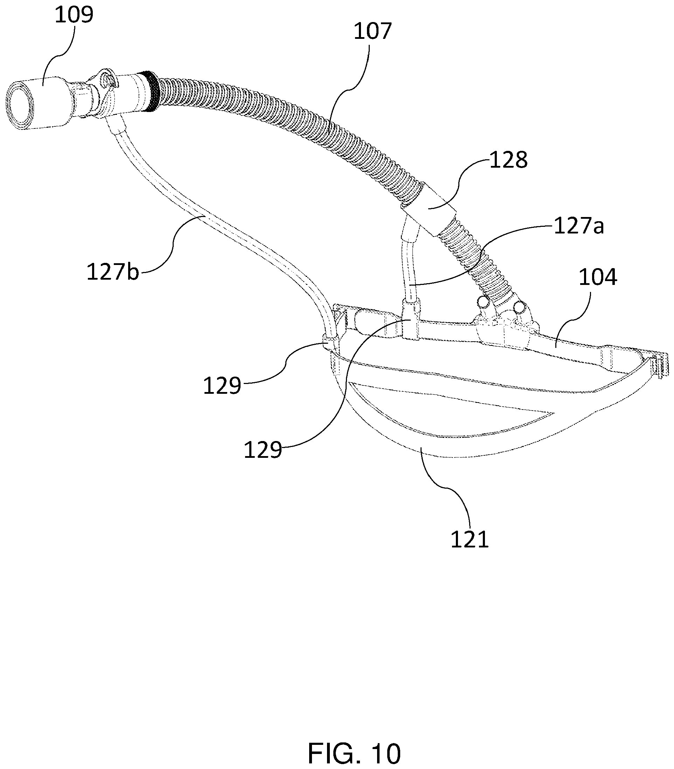

The supports 127a, 127b may be attached to the gas tube 107 at a common location, for example at a second connector 128 located on the gas tube 107, such as shown in Figure 9. In alternative examples the supports 127a, 127b may be attached to the gas tube 107 at different axial locations along the length of the gas tube 107, for example to two or more separate connectors 128 (not shown) or to part of the end connector 109. Figure 10 shows an example in which a first support 127a extends between the gas tube 107 and the strap 104, and a second support 127b extends between the end connector 109 and the patient head band 121.

The one or more first connectors 129 may be moveable on the strap 104, patient head band 121, or other suitable part of the nasal cannula 1. Alternatively, the first connectors 129 may be fixed in position, or may be selectively retained in a selected position on the nasal cannula 1. In some examples, the first connectors 129 may be temporarily detached from a first position on the nasal cannula 1 and reattached at a second position on the nasal cannula 1, for example from one side of the strap 104 to the other, or to part of the patient head band 121.

The one or more second connectors 128 may be moveable on the gas tube 107, or may be fixed on the gas tube 107, or may be selectively retained on the gas tube 107.

In the examples shown in Figures 9 and 10, the supports 127a, 127b are malleable supports that comprise malleable materials, such that the supports 127a, 127b retain a given shape or position when manipulated (e.g. bent or twisted). In alternative examples, the supports 127a, 127b may comprise a plurality of rigid members that extend between joints that retain a pivot angle when manipulated into said pivot angle. It will be clear to the skilled person that the examples described above may be adjusted in various ways.

The connector 8, 108 may be configured to vary its angle. For example, the elbow 8 may comprise a hinge mechanism. The hinge mechanism may be configured to allow the connector 8, 108 to move from a first position, configured to redirect gas flow at an angle of 30 degrees, to a second position, configured to redirect gas flow at an angle of 90 degrees. The hinge mechanism may be fixable at a plurality of angular positions.

In some examples, the connector 8 may not include an elbow. In this case, the gas tube 7, 107 may be coupled at one end to a straight connector pivotally connected to the manifold 2, 102 via the aperture 6, 106. Due to the flexibility of the gas tube 7, 107, the gas tube may form an elbow or at least provide some flexibility that can increase patient comfort and convenience whilst wearing the nasal cannula. The gas tube may have an elbow sleeve to form an elbow in the end of the gas tube nearest the manifold.

In the previous example, the connector is arranged to fit inside the aperture 6, 106. In alternative examples, a lip may extend from the aperture 6 such that the connector is arranged to fit over the lip.

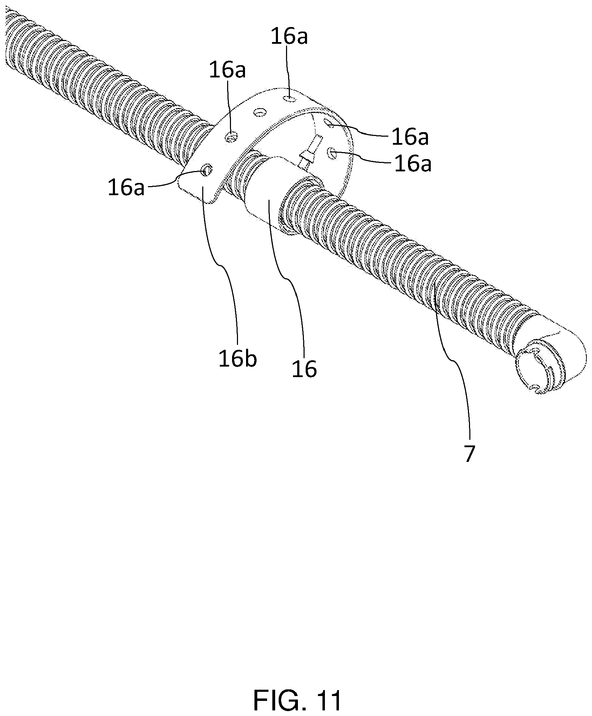

In some examples, for example as shown in Figure 11, the clip 16 may comprise a plurality of holes 16a (only some of the holes are labelled to increase clarity), with the strap portion 16b made correspondingly longer so that the size and tightness of the clip 16 can be adjusted. The nasal cannula 1, 101 may comprise a plurality of the clips 16, as required.

Although the invention has been described above with reference to one or more preferred embodiments, it will be appreciated that various changes or modifications may be made without departing from the scope of the invention as defined in the appended claims.

## Claims

1. A nasal cannula (1) configured to deliver airflow to a user, comprising:
a manifold (2);
a connector for attaching a gas tube (7) to the manifold;
a pair of non-sealing nasal prongs (3) extending from the manifold for delivering a supply of gas to a user via the gas tube;
wherein the connector is configured to form a swivel connection with the manifold so as to provide relative rotation between the manifold and the gas tube, **characterised in that**:
the swivel connection has an axis of rotation configured to be substantially normal to a face of the user; and
the nasal cannula further comprising a selective retainer means configured to selectively hold the connector in a plurality of rotary positions relative to the manifold.

2. A nasal cannula (1) according to claim 1, further comprising an elbow between the swivel connection and the gas tube (7), preferably, wherein the elbow is configured to redirect gas flow at an angle greater than 30 degrees, preferably greater than 60 degrees, and more preferably at substantially 90 degrees angle..

3. A nasal cannula (1) according to any preceding claim, wherein the swivel connection is configured to provide at least 120 degrees relative rotation, preferably at least 180 degrees and/or, wherein the swivel connection is configured to limit the relative rotation to an angle range less than 360 degrees, and preferably no more than 180 degrees.

4. A nasal cannula (1) according to any preceding claim, wherein a detent is used to releasably restrict or fix the rotary position.

5. A nasal cannula (1) according to any one of claims 1 to 3, wherein the selective retainer means is a friction fit between the connector and the manifold (2).

6. A nasal cannula (1) according to any preceding claim, wherein the connector forms a snap-fit connection with the manifold (2).

7. A nasal cannula (1) according to any preceding claim, wherein the connector attaches to the manifold (2) via an aperture (6) formed in the manifold.

8. A nasal cannula (1) according to claim 7, wherein the aperture (6) is located on the manifold (2) between opposing ends of the manifold, preferably wherein the aperture is centrally located on the manifold equidistant between opposing ends of the manifold.

9. A nasal cannula (1) according to claim 7 or 8, wherein the connector is configured to be inserted through the aperture (6).

10. A nasal cannula (1) according to any one of claims 7 to 9, wherein the manifold (2) comprises a face mount portion (110) configured to be placed proximate to the face of the user, and the aperture (6) is on an opposite side of the manifold to the face mount portion.

11. A nasal cannula (1) according to any preceding claim, wherein the manifold (2) comprises a rigid manifold structure (12) and a flexible manifold cover (13) configured to cover at least a portion of the rigid manifold structure.

12. A nasal cannula (1) according to claim 11, wherein the swivel connection has a first connection portion formed in the rigid manifold structure (12) and a second connection portion formed in the connector and/or wherein the non-sealing nasal prongs (3) are integrally formed with the flexible manifold cover (13).

13. A nasal cannula (1) according to any preceding claim, comprising a gas tube (7) connected to the connector, wherein the gas tube (7) is malleable or comprises a malleable member (117), such that the gas tube is configured to be deformable and retain a given shape when the gas tube is manipulated.

14. A system comprising:
a nasal cannula (1) according to any preceding claim; and
a gas supply configured to supply gas to a user via the gas tube (7).

15. A high flow oxygen therapy system according to claim 14.

## Patentansprüche

1. Nasenkanüle (1), ausgelegt zum Zuführen eines Luftstroms an einen Benutzer, umfassend:
einen Verteiler (2);
einen Verbinder zum Anbringen eines Gasschlauchs (7) an dem Verteiler;
ein Paar nichtdichtender Nasenbrillen (3), das sich von dem Verteiler wegerstreckt, zum Zuführen einer Gasversorgung an einen Benutzer über den Gasschlauch;
wobei der Verbinder ausgelegt ist, um eine Schwenkverbindung mit dem Verteiler und dem Gasschlauch zu bilden, **dadurch gekennzeichnet, dass**:
die Schwenkverbindung eine Drehachse aufweist, die ausgelegt ist, um im Wesentlichen normal zu einem Gesicht des Benutzers zu stehen; und
wobei die Nasenkanüle ferner ein selektives Halterungsmittel umfasst, das ausgelegt ist, um den Verbinder selektiv in einer Vielzahl von Drehpositionen relativ zum Verteiler zu halten.

2. Nasenkanüle (1) nach Anspruch 1, die weiters einen Winkelverbinder zwischen der Schwenkverbindung und dem Gasschlauch (7) umfasst, wobei der Winkelverbinder vorzugsweise ausgelegt ist, um einen Gasfluss in einem Winkel von mehr als 30°, vorzugsweise mehr als 60°, noch bevorzugter in einem Winkel von im Wesentlichen 90° umzuleiten.

3. Nasenkanüle (1) nach einem der vorangegangenen Ansprüche, wobei die Schwenkverbindung ausgelegt ist, um eine relative Drehung von zumindest 120°, vorzugsweise von zumindest 180° bereitzustellen und/oder wobei die Schwenkverbindung ausgelegt ist, um die relative Drehung auf einen Winkelbereich von weniger als 360° und vorzugsweise nicht mehr als 180° zu begrenzen.

4. Nasenkanüle (1) nach einem der vorangegangenen Ansprüche, wobei eine Arretierung verwendet wird, um die Drehposition lösbar zu begrenzen oder zu fixieren.

5. Nasenkanüle (1) nach einem der Ansprüche 1 bis 3, wobei das selektive Halterungsmittel eine Reibungspassung zwischen dem Verbinder und dem Verteiler (2) ist.

6. Nasenkanüle (1) nach einem der vorangegangenen Ansprüche, wobei der Verbinder eine Schnappverbindung mit dem Verteiler (2) bildet.

7. Nasenkanüle (1) nach einem der vorangegangenen Ansprüche, wobei der Verbinder über eine in dem Verteiler ausgebildete Öffnung (6) mit dem Verteiler (2) verbunden ist.

8. Nasenkanüle (1) nach Anspruch 7, wobei die Öffnung (6) zwischen gegenüberliegenden Enden des Verteilers auf dem Verteiler (2) angeordnet ist, wobei die Öffnung vorzugsweise mittig in gleichem Abstand zwischen gegenüberliegenden Enden des Verteilers angeordnet ist.

9. Nasenkanüle (1) nach Anspruch 7 oder 8, wobei der Verbinder ausgelegt ist, um durch die Öffnung (6) eingeführt zu werden.

10. Nasenkanüle (1) nach einem der Ansprüche 7 bis 9, wobei der Verteiler (2) einen Gesichtsbefestigungsabschnitt (110) umfasst, der ausgelegt ist, um in der Nähe des Gesichts des Benutzers platziert zu werden, und wobei sich die Öffnung (6) auf einer dem Gesichtsbefestigungsabschnitt des Verteilers entgegengesetzten Seite befindet.

11. Nasenkanüle (1) nach einem der vorangegangenen Ansprüche, wobei der Verteiler (2) eine starre Verteilerstruktur (12) und eine flexible Verteilerabdeckung (13) umfasst, die ausgelegt ist, um zumindest einen Abschnitt der starren Verteilerstruktur zu bedecken.

12. Nasenkanüle (1) nach Anspruch 11, wobei die Schwenkverbindung einen ersten Verbindungsabschnitt, der in der starren Verteilerstruktur (12) ausgebildet ist, und einen zweiten Verbindungsabschnitt aufweist, der in dem Verbinder ausgebildet ist, und/oder wobei die nichtdichtenden Nasenbrillen (3) einstückig mit der flexiblen Verteilerabdeckung (13) ausgebildet sind.

13. Nasenkanüle (1) nach einem der vorangegangenen Ansprüche, umfassend einen Gasschlauch (7), der mit dem Verbinder verbunden ist, wobei der Gasschlauch (7) formbar ist oder ein formbares Element (117) umfasst, sodass der Gasschlauch ausgelegt ist, um verformbar zu sein und eine gegebene Form behält, wenn der Gasschlauch verstellt wird.

14. System, das Folgendes umfasst:
eine Nasenkanüle (1) nach einem der vorangegangenen Ansprüche; und
eine Gaszufuhr, die ausgelegt ist, um über den Gasschlauch (7) Gas an einen Benutzer zuzuführen.

15. High-Flow-Sauerstoff-Therapiesystem nach Anspruch 14.

## Revendications

1. Canule nasale (1) configurée pour délivrer un flux d'air à un utilisateur, comprenant :
un collecteur (2) ;
un raccord pour fixer un tube à gaz (7) au collecteur ;
une paire de pinces nasales non scellantes (3) s'étendant à partir du collecteur pour délivrer une alimentation en gaz à un utilisateur via le tube à gaz ;
dans laquelle le raccord est configuré pour former une connexion pivotante avec le collecteur de manière à fournir une rotation relative entre le collecteur et le tube à gaz, **caractérisée en ce que** :
la connexion pivotante présente un axe de rotation configuré pour être sensiblement perpendiculaire à un visage de l'utilisateur ; et
la canule nasale comprenant en outre des moyens de retenue sélectifs configuré pour retenir sélectivement le raccord dans une pluralité de positions rotatives par rapport au collecteur.

2. Canule nasale (1) selon la revendication 1, comprenant en outre un coude entre la connexion pivotante et le tube à gaz (7), de préférence dans laquelle le coude est configuré pour rediriger le flux de gaz selon un angle supérieur à 30 degrés, de préférence supérieur à 60 degrés, et de manière plus préférée selon un angle sensiblement de 90 degrés.

3. Canule nasale (1) selon l'une quelconque des revendications précédentes, dans laquelle la connexion pivotante est configurée pour fournir une rotation relative d'au moins 120 degrés, de préférence d'au moins 180 degrés et/ou, dans laquelle la connexion pivotante est configurée pour limiter la rotation relative à une plage d'angle inférieure à 360 degrés, et de préférence ne dépassant pas 180 degrés.

4. Canule nasale (1) selon l'une quelconque des revendications précédentes, dans laquelle un cran est utilisé pour restreindre ou fixer de manière libérable la position rotative.

5. Canule nasale (1) selon l'une quelconque des revendications 1 à 3, dans laquelle les moyens de retenue sélectifs sont un ajustement par frottement entre le raccord et le collecteur (2).

6. Canule nasale (1) selon l'une quelconque des revendications précédentes, dans laquelle le raccord forme une connexion par encliquetage avec le collecteur (2).

7. Canule nasale (1) selon l'une quelconque des revendications précédentes, dans laquelle le raccord se fixe au collecteur (2) via une ouverture (6) formée dans le collecteur.

8. Canule nasale (1) selon la revendication 7, dans laquelle l'ouverture (6) est située sur le collecteur (2) entre des extrémités opposées du collecteur, de préférence dans laquelle l'ouverture est située au centre du collecteur à égale distance entre des extrémités opposées du collecteur.

9. Canule nasale (1) selon la revendication 7 ou 8, dans laquelle le raccord est configuré pour être inséré à travers l'ouverture (6).

10. Canule nasale (1) selon l'une quelconque des revendications 7 à 9, dans laquelle le collecteur (2) comprend une partie de monture de visage (110) configurée pour être placée à proximité du visage de l'utilisateur, et l'ouverture (6) est sur un côté opposé du collecteur par rapport à la partie de monture de visage.

11. Canule nasale (1) selon l'une quelconque des revendications précédentes, dans laquelle le collecteur (2) comprend une structure de collecteur rigide (12) et un couvercle de collecteur flexible (13) configuré pour couvrir au moins une partie de la structure de collecteur rigide.

12. Canule nasale (1) selon la revendication 11, dans laquelle la connexion pivotante présente une première partie de connexion formée dans la structure de collecteur rigide (12) et une seconde partie de connexion formée dans le raccord et/ou dans laquelle les pinces nasales non scellantes (3) sont formées d'un seul tenant avec le couvercle de collecteur flexible (13).

13. Canule nasale (1) selon l'une quelconque des revendications précédentes, comprenant un tube à gaz (7) relié au raccord, dans laquelle le tube à gaz (7) est malléable ou comprend un élément malléable (117), de telle sorte que le tube à gaz est configuré pour être déformable et conserver une forme donnée lorsque le tube à gaz est manipulé.

14. Système, comprenant :
une canule nasale (1) selon l'une quelconque des revendications précédentes ; et
une alimentation en gaz configurée pour fournir du gaz à un utilisateur via le tube à gaz (7).

15. Système d'oxygénothérapie à haut débit selon la revendication 14.
